# EUROPEAN PATENT SPECIFICATION

(11) **EP 1 406 091 B1**
(45) Date of publication and mention of the grant of the patent: **20.02.2008**
(21) Application number: 02738766.1
(22) Date of filing: 19.06.2002
(51) Int. Cl.: G01N 33/566, G01N 33/483, G01N 30/72, G01N 27/62, G01N 33/68, G01N 33/50

(54) **METHOD OF ANALYZING PROTEIN OCCURRING IN CELL OR SUBSTANCE INTERACTING WITH THE PROTEIN**
VERFAHREN ZUR ANALYSE EINES IN DER ZELLE AUFTRETENDEN PROTEINS ODER EINER MIT DEM PROTEIN WECHSELWIRKENDEN SUBSTANZ
METHODE D'ANALYSE D'UNE PROTEINE APPARAISSANT DANS UNE CELLULE OU D'UNE SUBSTANCE INTERAGISSANT AVEC LA PROTEINE

(30) Priority: 19.06.2001 JP 2001185602
(43) Date of publication of application: 07.04.2004
(73) Proprietor: SUNTORY LIMITED, Osaka-shi, Osaka 530-8203 (JP)
(72) Inventor: NAKAJIMA, Terumi, Tokyo 161-0031 (JP); NAOKI, Hideo, Sakai-shi, Osaka 592-5345 (JP); YASUDA, Akikazu, Osaka-shi, Osaka 531-0063 (JP); IWASA, Keiko, Kyoto-shi, Kyoto 607-8432 (JP)
(74) Representative: HOFFMANN EITLE
(86) International application number: PCT/JP2002/006103
(87) International publication number: WO 2002/103360

(56) References cited:
- WO-A-02/12899
- WO-A1-99/45150
- JP-A- 2001 249 125
- SHAH H N ET AL: "THE APPLICATION OF MATRIX-ASSISTED LASER DESORPTION/IONISATION TIME OF FLIGHT MASS SPECTROMETRY TO PROFILE THE SURFACE OF INTACT BACTERIAL CELLS" MICROBIAL ECOLOGY IN HEALTH & DISEASE, CHICHESTER, GB, vol. 12, no. 4, November 2000 (2000-11), pages 241-246, XP008030020
- LI L ET AL: "Single-cell MALDI: a new tool for direct peptide profiling" TRENDS IN BIOTECHNOLOGY, ELSEVIER, AMSTERDAM, NL, vol. 18, no. 4, April 2000 (2000-04), pages 151-160, XP004194003 ISSN: 0167-7799
- COURCHESNE: 'Identification of proteins by matrix-assisted laser desorption/ionization mass spectrometry using peptide and fragment ion masses' METHODS IN MOLECULAR BIOLOGY vol. 112, 1999, pages 487 - 511, XP002103063

## Description

### [TECHNICAL FIELD]

This invention relates to a method for evaluating, identifying, and structurally analyzing substances interacting with proteins present in cells of organisms, and also relates to a method for determining the presence of particular proteins in cells of organisms by use of substances interacting with the proteins.

### [BACKGROUND ART]

On the superficial layer of cells or in cells (herein, both situations may be simply expressed as "in cells" collectively), various proteins are known to exist which play various roles in a living organism, such as a role as receptors, a role as ion channels, a role as transporters, a role as antibodies, a role as enzymes, and a role involved in intracellular signal transduction. It is also known that there are numerous substances which interact with these proteins to activate or inhibit the functions of these proteins, or to act as antigens for the antibodies, or to work as substrates for the enzymes.

However, not all has been elucidated about such proteins present in cells of organisms or such substances interacting with the proteins. Even nowadays, searches for new proteins, or for substances interacting with various intracellular proteins, have been conducted energetically by researchers all over the world.

To search the natural world for novel physiologically active substances interacting with particular proteins, bioassays using proteins with known activities have hitherto been employed generally. However, bioassays have included at least two problems. First, materials are consumed during purification of physiologically active substances, so that large amounts of the starting ingredient and naturally occurring matter (including organisms) for extraction of the ingredient are needed to secure sufficient amounts of materials for structural determination. Secondly, available bioassays are limited and, with the standard bioassays, many novel physiologically active substances may remain undetected.

Recently, matrix-assisted laser desorption/ionization time-of-flight mass spectrometry (MALDI-TOF MS) has become a powerful tool for the direct analysis of peptide profiles from small pieces of dissected tissues or from isolated single cells (Garden et al., 1996; De With et al., 1997; Garden et al., 1998; Redeker et al., 1998). In these experiments, it is possible to analyze very fine regions of samples, and the resulting peptide fingerprinting shows the biosynthesis and expression of bioactive peptides.

WO 02/12899 discloses the analysis of peptides generated from a protein by a cell, which peptides are bound to cell surface proteins, in particular MHC molecules.

WO 99/45150 discloses methods for analyzing the interaction between ligands and biomolecular targets. MALDI-TOF is mentioned as a suitable technique for studying large biomolecules.

Shah et al., 2000, Microbial Ecology in Health and Disease, 12: 241-246, relates to analysis of different bacteria strains by use of MALDI-TOF-MS on the basis of species characteristic markers.

Li et al., 2000, TIBTECH, 18: 151-159, discusses the use of single-cell MALDI as a tool for direct peptide profiling. According to this document MALDI-MS can profile the peptides and proteins from single-cell and small tissue samples without the need for extensive sample preparation, except for the cell isolation and matrix application.

On the other hand, on-line capillary reversed-phase high performance liquid chromatography with mass spectrometric detection (capillary HPLC/MS and capillary HPLC/MS/MS) is available in modern bioanalysis. This tool provides excellent information on the molecular weights of peptides in samples, and the MS/MS system allows the peptides to be fragmented to yield fragment ions, enabling their amino acid sequences to be deduced.

There has been a desire for the development of a new method for screening substances interacting with proteins present in cells, and a new method for determining the presence of particular proteins in cells with the use of substances interacting with the proteins, the new methods not relying on conventional bioassays. As these methods, methods easy to operate and capable of accurate and prompt determination have been desired.

### [DISCLOSURE OF THE INVENTION]

We, the inventors of the present invention, already developed a method for directly identifying the chemical structure of a substance present in tissue or cells of organisms by use of MALDI-TOF MS, and filed an application for it as Japanese Patent Application No. 2000-060754. The present invention has been accomplished as a result of in-depth studies with a view to expanding the range of usage of this application. The present invention has succeeded in developing a method for determining the presence of a protein, which is present on a cell membrane or in cells, or the presence of a substance interacting with this protein, by the use of the protein and the substance.

### [BRIEF DESCRIPTION OF THE DRAWINGS]

FIG. 1 is a chart showing the results of MALDI-TOF-MS measurement of a ligand(hANP)-bound membrane fraction prepared with the use of cells having expressed GC-A, receptors of hANP, in Example 1.
FIG. 2 is a chart showing the results of MALDI-TOF-MS measurement of a ligand(hANP)-bound membrane fraction prepared with the use of blank cells free from expression of GC-A in Example 1.
FIG. 3 is a chart showing the results of MALDI-TOF-MS measurement of a ligand-bound membrane fraction prepared with the use of a ligand (a peptide mixture solution comprising four peptides including hANP) and GC-A expressed cells in Example 2.
FIG. 4 is a chart showing the results of MALDI-TOF-MS measurement of a ligand-bound membrane fraction prepared with the use of a ligand (a peptide mixture solution comprising four peptides including hANP) and blank cells in Example 2.
FIG. 5 is a chart showing the results of MALDI-TOF-MS measurement of only the peptide mixture solution used in Example 2.

### [EMBODIMENTS OF THE INVENTION]

A first embodiment of the method according to the present invention is that if a known protein is present on a cell membrane or in cells, the cells and an unknown test substance are brought into contact, causing the test substance to interact with the cells in some form, such as adsorption, binding or association. In this state, the cell or a slice of the cell (for example, a cell membrane) is purified, and the amount of the test substance present is confirmed by the use of MALDI-TOF MS. By this procedure, whether the test substance interacts with the known protein can be judged.

A second embodiment of the method according to the present invention is a method which is contrary to the first embodiment, and is the method which uses a known substance, for example, a ligand to a particular receptor, and judges whether an unknown protein present on a cell membrane or in cells is existent or not. In this case, the cells having the unknown protein are brought into contact with the known substance. If the known substance has interacted with the cells in some form, such as adsorption, binding or association, it can be judged that the protein, which the known substance acts on, is present on the cell membrane or in the cells.

Furthermore, the amino acid sequence of the protein, whose existence has been confirmed, can be determined by the method of the present invention.

According to the present invention, ligands, which act on various receptors expressed on the nuclear membrane and mitochondrial membrane as well as the cell membrane, are purified while being adsorbed to the membrane, and the ligand-bound cell membrane is irradiated with a laser beam. By this procedure, only the ligand can be directly ionized, and the ligand can be analyzed by mass spectrometry.

Examples of the receptors are peptide hormone receptors, ion channels, transporters and, further, antibodies expressed in lymphocytes and macrophages. The ligands, on the other hand, include not only various naturally occurring physiologically active substances, such as peptides, amino acids, amines, and steroids, but also synthetic compounds working as ligands. Thus, the present method is effective as a means of searching for unknown ligands interacting with such receptors, and can be used to search for receptors on the cell membrane which have unknown functions.

Mass spectrometry for use in the analysis system can make an analysis with a cell membrane fraction corresponding to 10⁴ to 10⁵ cells, for example, in the case of cells, or with a very small amount of a sample measuring 2 mm × 2 mm × 0.1 mm in thickness, for example, in the case of a tissue slice. Also, the mass spectrometry method can obtain, simultaneously and in a short time, molecular information on the molecular weight, the types of molecules (types, such as peptide proteins, steroids, lignans, catechins, and sugars; hereinafter referred to as "chemical species"), and the structure of molecules. That is, the use of the MALDI-TOF model as a mass spectrometer results in the measurement of the molecular weight of the ligand. Moreover, the full use of a µ-column LC-ESI-QTOF-MS/MS analyzer, with its molecule related ions as precursor ions, enables the chemical species of adsorbed molecules and even their structure to be analyzed.

By combining these methods, the presence of novel physiologically active substances, or receptors expressed in cultured cells from tissue, can be predicted. These methods can contribute, particularly, to the discovery of orphan receptors and ligands thereto, and are also useful as a means of screening new medicines, or are useful for searching for disease markers.

According to the present invention, moreover, the method of the invention can be applied widely not only to combinations of receptors and ligands, but also to substances interacting with proteins in some form. These interactions include, for example, actions on proteins which are ion channels, transporters, antibodies, and enzymes. Substances having such actions include antigens to antibodies, substrates to enzymes, or activators or inhibitors.

The present invention will be described with reference, for example, to an embodiment in which a ligand to cells expressing a known receptor is detected and identified.

Known cells expressing known receptors are cultured to express the receptors. The cultured cells are used as sample cells, and treated in the manner described below. For comparison, the same type of cells which have not expressed the receptors are used. The present invention can utilize not only cultured cells expressing such known receptors, but also tissue cells from organ tissues, and dissociated cells such as lymphocytes and intraperitoneal monocytes.

Distilled water in a proper amount, for example, in an amount of 100 to 800 µl, is added to a suitable number, for example, 10³ to 10⁵, preferably 10⁴ to 10⁵, of the sample cells. The mixture is incubated at room temperature for 3 to 30 minutes, preferably 5 to 20 minutes, to crush the cells. The system is centrifuged to precipitate the cell membranes, or the system is filtered to separate the cell membranes. The cell membranes are washed with distilled water, and centrifuged or filtered again to remove the cytoplasm, thereby obtaining a receptor-expressed cell membrane fraction.

To the resulting membrane fraction, a solution of a substance, which is enough to coat a nonspecific adsorption portion of the membrane fraction and which has the effect of hindering the nonspecific adsorption of a substance, for example, 1 pmol/µl of albumin (BSA), is added in an amount of 200 to 500 µl, and the system is dispersed. The dispersion is incubated at room temperature for 3 to 30 minutes, preferably 5 to 20 minutes to coat the nonspecific adsorption portion of the membrane fraction with albumin. The membrane fraction is gathered by centrifugation or filtration, and washed with distilled water to remove the excess albumin.

A ligand in a sufficient amount to bind to the receptors, for example, 100 µl of a 10 pmol/µl ligand solution, is added to the washed albumin-coated membrane fraction, and the mixture is incubated at room temperature for 3 to 30 minutes, preferably 5 to 20 minutes. Then, the system is subjected to a washing treatment involving washing with distilled water, followed by centrifugation (or filtration). This washing treatment is repeated two times or more, preferably 4 to 5 times, to remove fractions which have not adsorbed the excess ligand.

A proper amount, for example, 5 to 20 µl, of distilled water is added to the resulting ligand-adsorbed cell membranes, and the system is suspended by a voltex. The suspension (0.5 to 3 µl) is dispensed into a plate for MALDI, and air-dried. An α-cyano-4-hydroxycinnamic acid (αCHCA) matrix solution is added to the air-dried suspension, and MALDI-TOF-MS measurement is made. This measurement is made similarly for the comparative cell membrane which has not expressed the receptors. The results of these measurements are compared, whereby the ligand bound to the known receptors can be identified, with the molecular weight as an indicator.

According to the present invention, the full use of a µ-column LC-ESI-QTOF-MS/MS meter - using laser-ionized molecule related ions, which have been used for mass spectrometry, as precursor ions - enables the chemical species of the adsorbed molecules and even their structure to be analyzed.

According to the present invention, a protein present on the superficial layer of cells or in cells, or a substance interacting with the protein can be detected, identified or analyzed by the steps described below.

The above object can be achieved by a method comprising:
(1) the step of bringing cells into contact with a substance interacting with a protein on the superficial layer of the cells or in the cells to cause the substance to interact with the cells;
(2) the step of purifying the cells, with the cells and the substance being in an interacting state;
(3) the step of irradiating the purified cells with a laser, with the substance in the interacting state, to ionize the substance directly; and
(4) the step of analyzing the ionized substance by mass spectrometry.

The present invention can also screen a substance interacting with a protein present on the superficial layer of cells or in cells, the protein having known activity, by a method comprising the steps described below.

A mass spectrum obtained by
(1) the step of bringing a test substance into contact with cells, where a known protein is known to be present, to cause interaction;
(2) the step of purifying the cells having interacted with the test substance, or a fraction thereof;
(3) the step of irradiating the purified cells or the fraction thereof with a laser, thereby directly ionizing the substance having interacted with the cells; and
(4) the step of analyzing the ionized substance by mass spectrometry,
is compared with a mass spectrum obtained by performing the steps (1) to (4) with the use of cells where the protein has been found not to be present. If the former mass spectrum shows that the amount of the test substance increases, it can be judged that the test substance is a substance interacting with the protein. The identification of the substance makes it possible to screen and identify the substance interacting with the known protein present on the superficial layer of cells or in cells.

Furthermore, according to the present invention, expression cells or expression tissue for receptors interacting with a known ligand, for example, can be screened by the method described below.

This method comprises comparing a mass spectrum, which has been obtained by
(1) the step of bringing a known ligand into contact with tissue or cells, where the presence or absence of receptors to the ligand is to be confirmed, to cause interaction;
(2) the step of purifying the tissue or cells contacted with the ligand;
(3) the step of irradiating the purified tissue or cells with a laser, thereby directly ionizing the ligand present; and
(4) the step of analyzing the ionized ligand by mass spectrometry,
with a mass spectrum obtained by performing only the step (2) and the subsequent steps, without performing step (1) in which the known ligand is brought into contact with the tissue or cells. If the former mass spectrum shows that the amount of the ligand increases, it can be judged that the receptors interacting with the ligand are present in the tissue or cells.

The present invention will be described in further detail with reference to the Examples offered below.

### [Example 1] Experiments on binding between human atrial natriuretic peptide (hereinafter referred to as "hANP") and hANP receptor GC-A

Cultured cells were receptor expressed cells and blank cells. CHO/GC-A, i.e. CHO cells (Chinese hamster ovary cells) having expressed hANP receptor GC-A, were used as the receptor expressed cells. CHO host cells were used as the blank cells. MALDI-TOF-MS measurement samples were prepared by the same method in the following manner:

### Step 1. Preparation of membrane fraction

Distilled water (500 µl) was added to 7.5×10⁵ cultured cells (GC-A expressed cells and blank cells), and the mixture was incubated at room temperature for 10 minutes. The resulting suspension was centrifuged to obtain pellets. Distilled water (500 µl) was further added to the pellets, and the mixture was centrifuged again, followed by removing the supernatant, to obtain a membrane fraction.

### Step 2. Albumin coating

To the resulting membrane fraction, 500 µl of a 1 pmol/µl aqueous albumin solution was added, and the mixture was incubated for 10 minutes at room temperature. The resulting suspension was centrifuged to obtain pellets. Distilled water (500 µl) was further added to the pellets, and the mixture was centrifuged again to obtain an albumin-coated membrane fraction.

### Step 3. Binding to ligand (hANP)

To the resulting albumin-coated membrane fraction, 100 µl of a 10 pmol/µl aqueous hANP solution was added, and the mixture was incubated for 10 minutes at room temperature. The resulting suspension was centrifuged to obtain pellets. The resulting pellets were further washed with 500 µl of distilled water, and centrifugation was performed again to obtain a ligand(hANP)-bound membrane fraction.

### Step 4. MALDI-TOF-MS measurement

Distilled water (10 µl) was added to the resulting ligand-bound membrane fraction, and the mixture was suspended. The suspension (1 µl) was dispensed into a plate for analysis, and air-dried. Then, 1 µl of an α-cyano-4-hydroxycinnamic acid (α-CHCA) saturated solution (methanol:water = 1:1, 0.1% trifluoroacetic acid) was added to the air-dried suspension, and MALDI-TOF-MS measurement (Voyager Elite) was made.

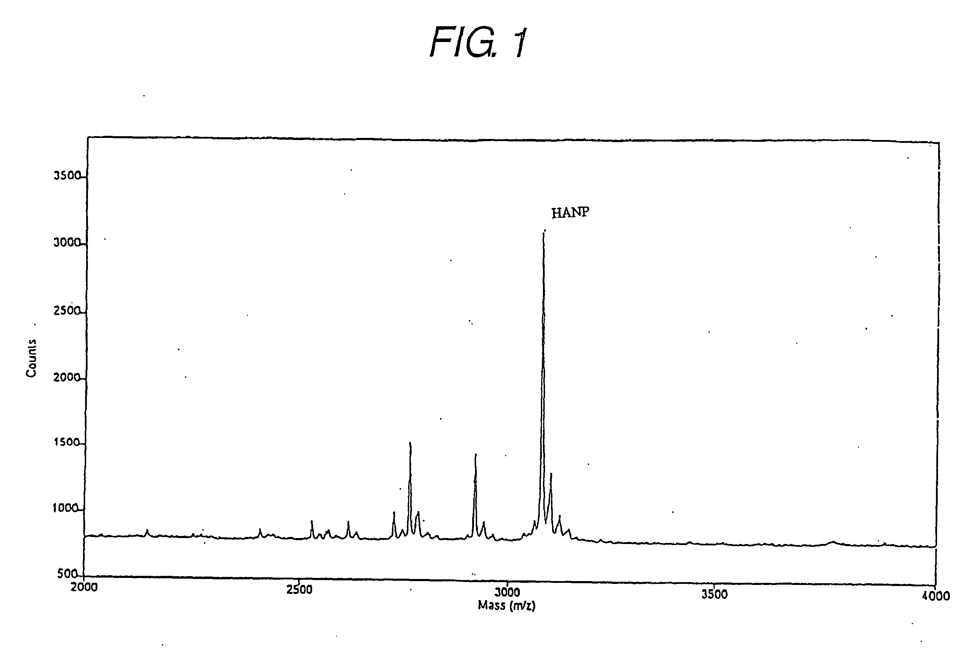

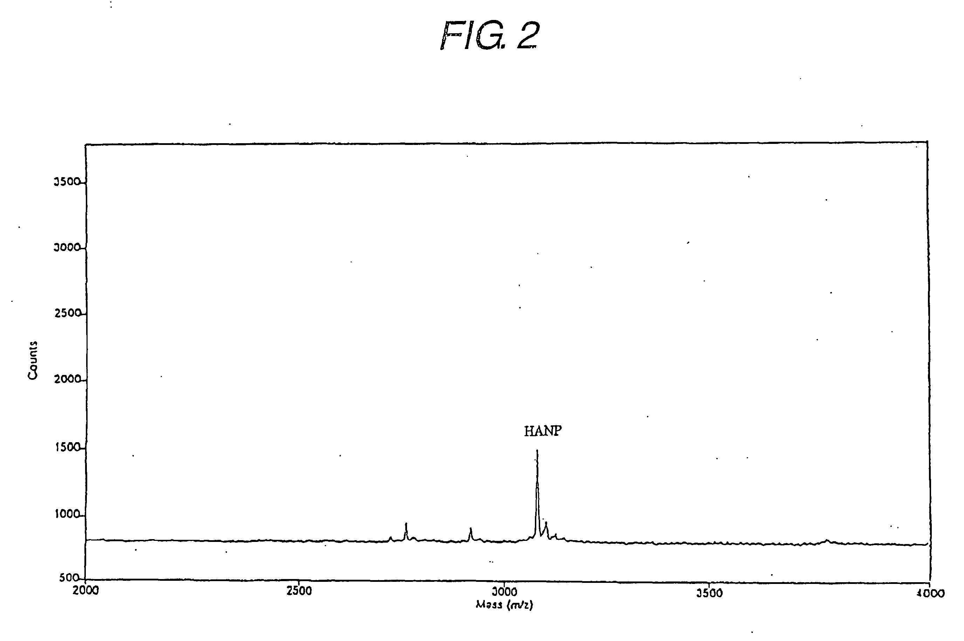

As shown in FIGS. 1 and 2, hANP detected was about three times as much in the GC-A expressed cells (FIG. 1) as in the blank cells (FIG. 2). From these findings, hANP can be judged to interact with GC-A receptors.

### [Example 2] Experiments on selectivity

### Step 1. Preparation of membrane fraction and

### Step 2. Albumin coating

An albumin-coated membrane fraction (CHO/GC-A cells and CHO host cells) was obtained in the same manner as in Step 1. and Step 2. of Example 1.

### Step 3. Binding to ligand (peptide mixture)

To the resulting albumin-coated membrane fraction, 100 µl of a peptide mixture solution, i.e., an aqueous solution of a mixture of 10 pmol/µl of hANP, 10 pmol/µl of angiotensin I, 10 pmol/µl of substance P (SP), and 10 pmol/µl of melanocyte-stimulating hormone (α-MSH), was added, and the mixture was incubated for 10 minutes at room temperature. The resulting suspension was centrifuged to obtain pellets. The resulting pellets were further washed with 500 µl of distilled water, and centrifugation was performed again to obtain a ligand-bound membrane fraction.

### Step 4. MALDI-TOF-MS measurement

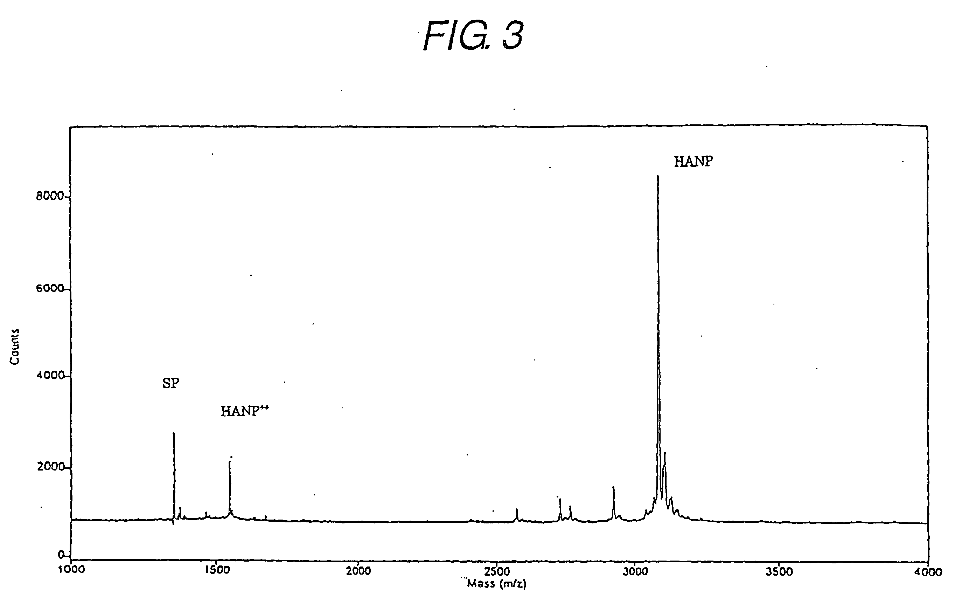

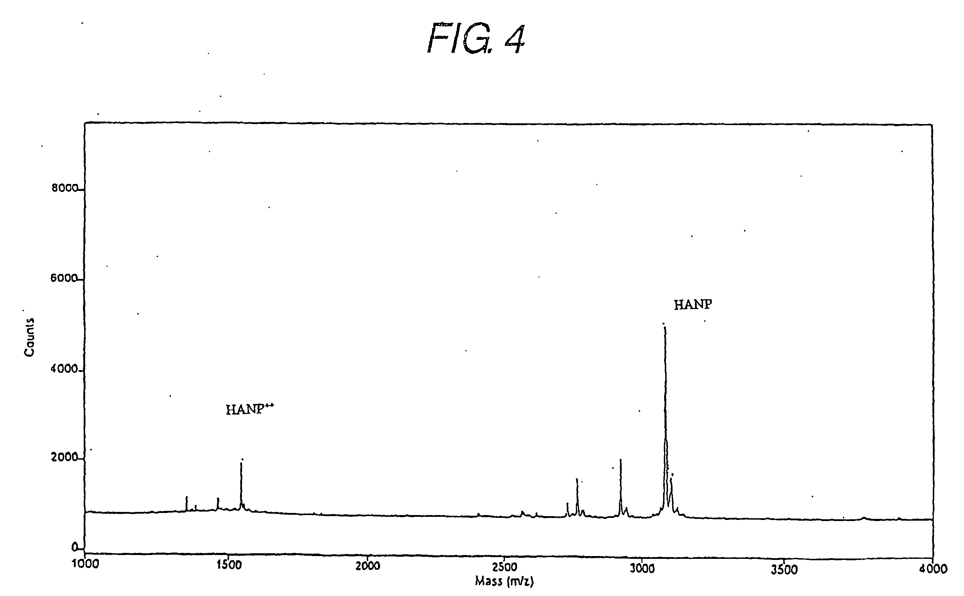

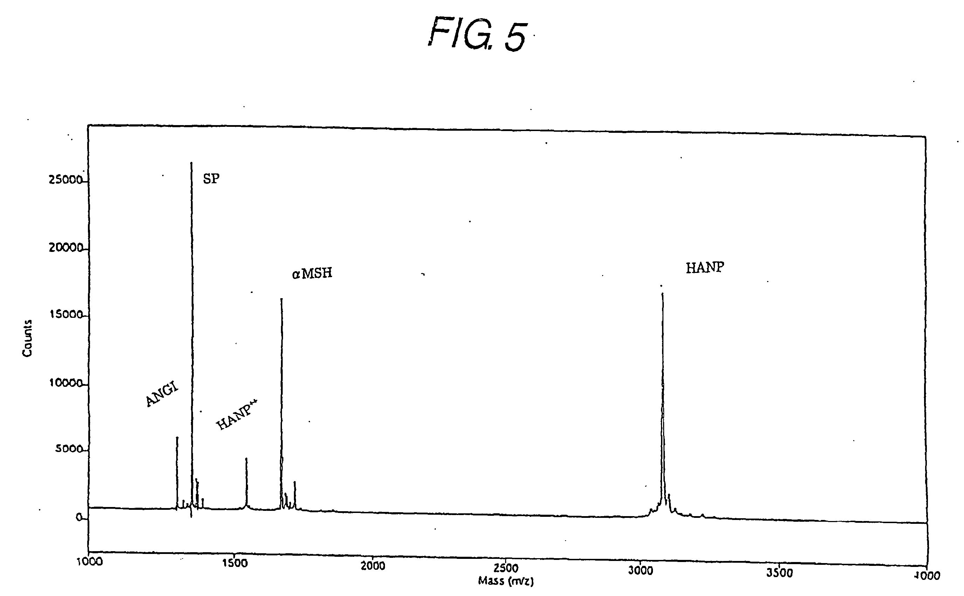

MALDI-TOF-MS measurement was made in the same manner as in Step 4. of Example 1. The results are shown in FIG. 3 (GC-A receptors were expressed) and FIG. 4 (the blank without expression of GC-A receptors). For comparison, MALDI-TOF-MS measurement was made only of 1 µl of the peptide mixture solution (10 pmol/µl of hANP, 10 pmol/µl of angiotensin I, 10 pmol/µl of SP, and 10 pmol/µl of α-MSH) (FIG. 5).

It was found that hANP was bound to the GC-A receptor expressed cell membrane fraction 5 or more times as selectively as the three other peptides. Thus, it can be judged that only hANP interacts with GC-A receptors, and the other peptides, i.e. angiotensin I, substance P and α-MSH, do not interact with GC-A receptors.

### [INDUSTRIAL APPLICABILITY]

According to the method of the present invention, if a known protein is present on a cell membrane or in cells, the cells and an unknown test substance are brought into contact, causing the test substance to interact with the cells in some form, such as adsorption, binding or association. In this state, the cells or slices of the cell (for example, cell membranes) are purified, and the amount of the test substance present is confirmed by the use of MALDI-TOF MS. By this procedure, whether the test substance interacts with the known protein can be judged.

Another embodiment of the present invention is a method which is contrary to the above method, and which uses a known substance, for example, a ligand to a particular receptor, and judges whether an unknown protein present on a cell membrane or in cells is existent or not. In this case, the cells having the unknown protein are brought into contact with the known substance. If the known substance has interacted with the cells in some form, such as adsorption, binding or association, it can be judged that the protein, which the known substance acts on, is present on the cell membrane or in the cells. Furthermore, the amino acid sequence of the protein, whose existence has been confirmed, can be determined by the method of the present invention.

These methods are easy to operate, and capable of accurate and prompt judgment. Thus, a wide range of applications can be expected of them.

## Claims

1. A method for identifying or analyzing a protein present on a superficial layer of cells or in cells, or a substance interacting with said protein, comprising:
(1) a step of bringing the cells having said protein and said substance interacting with said protein into contact with each other to cause said substance to bind to the protein;
(2) a step of purifying the cells, with the protein and said substance being in a bound state;
(3) a step of irradiating the purified cells with a laser, with said substance being in the bound state, to ionize said substance directly; and
(4) a step of analyzing the ionized substance by matrix-assisted laser desorption/ionization time-of-flight mass spectrometry (MALDI-TOF MS).

2. The method according to claim 1, wherein said protein present on the superficial layer of cells or in the cells is a protein showing a physiological activity as a receptor or an antibody.

3. The method according to claim 1, wherein said substance interacting with said protein is a ligand, an antigen or a substrate to said protein.

4. The method according to claim 1, wherein said substance interacting with said protein is a substance present in a living organism.

5. The method according to claim 4, wherein said substance present in a living organism has a molecular weight of 500 to 3,000.

6. The method according to claim 1, wherein said substance interacting with said protein is a substance not present in a living organism.

7. The method according to claim 1, wherein said mass spectrometry is a method using a matrix-assisted laser desorption/ionization time-of-flight mass spectrometer (MALDI-TOF MS) and a microcolumn liquid chromatography electrospray ionization quadrupole time-of-flight tandem mass spectrometer (µ-column LC-ESI-QTOF-MS/MS).

8. A method for screening a substance interacting with a protein present on a superficial layer of cells or in cells, said protein having known activity, said method comprising:
comparing a mass spectrum obtained by
(1) a step of bringing a test substance into contact with cells, where said protein is known to be present, to bind said test substance to said protein,
(2) a step of purifying the cells, with the cells and said test substance being in a bound state,
(3) a step of irradiating the purified cells with a laser, thereby directly ionizing said substance bound to the cells, and
(4) a step of analyzing the ionized substance by matrix-assisted laser desorption/ionization time-of-flight mass spectrometry (MALDI-TOF MS),
with a mass spectrum obtained by performing the steps (1) to (4) with use of cells where said protein has been known to be absent, whereby presence of said substance bound to said protein is determined.

9. The method according to claim 8, wherein said known protein is a receptor protein.

10. The method according to claim 9, wherein an isolated and established strain is used as the cells in which said receptor protein is expressed.

11. The method according to claim 9, wherein cells artificially forced to express said receptor protein are used as the cells in which said receptor is expressed.

12. The method according to claim 9, wherein orphan receptor expressing cells are used as the cells in which said receptor protein is expressed.

13. A method for screening cells or tissue, in which a protein interacting with a substance with known activity, is expressed, comprising:
comparing a mass spectrum obtained by
(1) a step of bringing said substance into contact with cells or tissue, where presence or absence of said protein is to be confirmed, to bind said substance to said protein,
(2) a step of purifying the cells or tissue binding said substance,
(3) a step of irradiating the purified cells or tissue with a laser, thereby directly ionizing said substance present, and
(4) a step of analyzing the ionized substance by matrix-assisted laser desorption/ionization time-of-flight mass spectrometry (MALDI-TOF MS),
with a mass spectrum obtained by performing only the steps (2) to (4), without performing step (1) in which the ligand is brought into contact with said cells or tissue , whereby presence of said protein interacting with said substance is determined.

14. The method according to claim 13, wherein said substance is a ligand.

15. The method according to claim 13 or 14, wherein said protein is a receptor protein.

## Patentansprüche

1. Verfahren zum Identifizieren oder Analysieren eines Proteins, das auf einer oberflächlichen Schicht von Zellen oder in Zellen vorhanden ist, oder einer Substanz, die mit diesem Protein interagiert, umfassend:
(1) einen Schritt des Inkontaktbringens der Zellen mit diesem Protein und dieser Substanz, die mit diesem Protein interagiert, miteinander, um diese Substanz zu veranlassen, an das Protein zu binden;
(2) einen Schritt des Reinigens der Zellen, wobei das Protein und diese Substanz in einem gebundenen Zustand sind;
(3) einen Schritt des Bestrahlens der gereinigten Zellen mit einem Laser, wobei diese Substanz in einem gebundenen Zustand vorliegt, um diese Substanz direkt zu ionisieren; und
(4) einen Schritt des Analysierens der ionisierten Substanz durch matrixassistierte Laserdesorption/Ionisierung-Flugzeit-Massenspektrometrie (MALDI-TOF MS).

2. Verfahren gemäß Anspruch 1, wobei das Protein, das auf der oberflächlichen Schicht von Zellen oder in den Zellen vorhanden ist, ein Protein ist, das eine physiologische Aktivität als Rezeptor oder Antikörper zeigt.

3. Verfahren gemäß Anspruch 1, wobei die Substanz, die mit diesem Protein interagiert, ein Ligand, ein Antigen oder ein Substrat dieses Proteins ist.

4. Verfahren gemäß Anspruch 1, worin die Substanz, die mit diesem Protein interagiert, eine Substanz ist, die in einem lebenden Organismus vorhanden ist.

5. Verfahren gemäß Anspruch 4, wobei die Substanz, die in einem lebenden Organismus vorhanden ist, ein Molekulargewicht von 500 bis 3.000 hat.

6. Verfahren gemäß Anspruch 1, wobei die Substanz, die mit dem Protein interagiert, eine Substanz ist, die nicht in einem lebenden Organismus vorhanden ist.

7. Verfahren gemäß Anspruch 1, wobei die Massenspektrometrie ein Verfahren ist, das matrixassistierte Laserdesorption/Ionisierung-Flugzeit-Massenspektrometrie (MALDI-TOF MS) und ein Mikrosäulen-Flüssigchromatographie-Elektrospray-Ionisierung-Vierfach-Flugzeit-Tandem-Massenspektrometer (µ-Säule LC-ESI-QTOF-MS/MS) ist.

8. Verfahren zum Durchmustern einer Substanz, die mit einem Protein interagiert, welches auf einer oberflächlichen Schicht von Zellen oder in Zellen vorhanden ist, wobei das Protein eine bekannte Aktivität hat und das Verfahren umfaßt:
Vergleichen eines Massenspektrums, das gewonnen wurde durch
(1) einen Schritt des Inkontaktbringens einer Testsubstanz mit Zellen, wobei das Protein bekanntermaßen vorhanden ist, um diese Testsubstanz an das Protein zu binden,
(2) einen Schritt des Reinigens der Zellen mit den Zellen und der Testsubstanz in gebundenem Zustand,
(3) einen Schritt des Bestrahlens der gereinigten Zellen mit einem Laser und dadurch das direkte Ionisieren dieser Substanz, die an die Zellen gebunden ist, und
(4) einen Schritt des Analysierens der ionisierten Substanz durch matrixassistierte Laserdesorption/Ionisierung-Flugzeit-Massenspektrometrie (MALDI-TOF MS),
mit einem Massenspektrum, das durch Durchführen der Schritte (1) bis (4) gewonnen wurde, unter Verwendung von Zellen, in denen das Protein bekanntermaßen nicht vorhanden war, wobei das Vorhandensein dieser Substanz, welche an das Protein bindet, bestimmt wird.

9. Verfahren gemäß Anspruch 8, wobei dieses bekannte Protein ein Rezeptorprotein ist.

10. Verfahren gemäß Anspruch 9, wobei ein isolierter und etablierter Stamm als Zellen verwendet wird, in denen dieses Rezeptorprotein exprimiert wird.

11. Verfahren gemäß Anspruch 9, wobei die Zellen, die künstlich dazu gezwungen werden dieses Rezeptorprotein zu exprimieren, als Zellen verwendet werden, in denen dieser Rezeptor exprimiert wird.

12. Verfahren gemäß Anspruch 9, wobei Orphan-Rezeptor exprimierende Zellen als Zellen verwendet werden, in denen das Rezeptorprotein exprimiert wird.

13. Verfahren zum Durchmustern von Zellen oder Gewebe, in denen ein Protein, das mit einer Substanz mit bekannter Aktivität interagiert, exprimiert wird, umfassend:
Vergleichen eines Massenspektrums, das gewonnen wurde durch
(1) einen Schritt des Inkontaktbringens dieser Substanz mit Zellen oder Gewebe, wobei das Vorhandensein oder die Abwesenheit dieses Proteins zu bestätigen ist, um diese Substanz an dieses Protein zu binden,
(2) einen Schritt des Reinigens der Zellen oder des Gewebes, das diese Substanz bindet,
(3) einen Schritt des Bestrahlens der gereinigten Zellen oder des Gewebes mit einem Laser und dadurch das direkte Ionisieren der vorhandenen Substanz, und
(4) einen Schritt des Analysierens der ionisierten Substanz durch matrixassistierte Laserdesorption/Ionisierung-Flugzeit-Massenspektrometrie (MALDI-TOF MS),
mit einem Massenspektrum, das ohne Durchführung von Schritt (1) alleine durch Durchführen der Schritte (2) bis (4) gewonnen wurde, wobei der Ligand mit diesen Zellen oder Gewebe in Kontakt gebracht wird, wodurch das Vorhandensein dieses Proteins, das mit dieser Substanz interagiert, bestimmt wird.

14. Verfahren gemäß Anspruch 13, wobei die Substanz ein Ligand ist.

15. Verfahren gemäß Anspruch 13 oder 14, wobei das Protein ein Rezeptorprotein ist.

## Revendications

1. Procédé d'identification ou d'analyse d'une protéine présente sur une couche superficielle de cellules ou dans des cellules, ou d'une substance interagissant avec ladite protéine, comprenant :
(1) une étape de mise en contact les cellules ayant ladite protéine et ladite substance interagissant avec ladite protéine les unes avec les autres pour provoquer la liaison de ladite substance à la protéine ;
(2) une étape de purification des cellules, avec la protéine et ladite substance étant dans un état lié ;
(3) une étape d'irradiation des cellules purifiées avec un laser, avec ladite substance étant dans l'état lié, pour ioniser ladite substance directement ; et
(4) une étape d'analyse de la substance ionisée par spectrométrie de masse à temps de vol avec désorption- ionisation laser assistée par matrice (MALDI-TOF-MS).

2. Procédé selon la revendication 1, dans lequel ladite protéine présente sur la couche superficielle des cellules ou dans les cellules est une protéine présentant une activité physiologique sous la forme d'un récepteur ou d'un anticorps.

3. Procédé selon la revendication 1, dans lequel ladite substance interagissant avec ladite protéine est un ligand, un antigène ou un substrat pour ladite protéine.

4. Procédé selon la revendication 1, dans lequel ladite substance interagissant avec ladite protéine est une substance présente dans un organisme vivant.

5. Procédé selon la revendication 4, dans lequel ladite substance présente dans un organisme vivant a un poids moléculaire de 500 à 3000.

6. Procédé selon la revendication 1, dans lequel ladite substance interagissant avec ladite protéine est une substance non présente dans un organisme vivant.

7. Procédé selon la revendication 1, dans lequel ladite spectrométrie de masse est un procédé utilisant un spectromètre de masse à temps de vol avec désorption-ionisation laser assistée par matrice (MALDI-TOF-MS) et un spectromètre de masse en tandem à temps de vol quadripolaire avec ionisation par électronébulisation et chromatographie liquide sur microcolonne (µ-colonne LC-ESI-QTOF-MS/MS).

8. Procédé de criblage d'une substance interagissant avec une protéine présente sur une couche superficielle de cellules ou dans des cellules, ladite protéine possédant une activité connue, ledit procédé comprenant :
la comparaison d'un spectre de masse obtenu par
(1) une étape de mise en contact d'une substance à tester avec des cellules, où ladite protéine est connue pour être présente, pour lier ladite substance à tester à ladite protéine,
(2) une étape de purification des cellules, avec les cellules et ladite substance à tester étant dans un état lié,
(3) une étape d'irradiation des cellules purifiées avec un laser, de cette manière ionisant directement ladite substance liée aux cellules, et
(4) une étape d'analyse de la substance ionisée par spectrométrie de masse à temps de vol avec désorption-ionisation laser assistée par matrice (MALDI-TOF-MS),
avec un spectre de masse obtenu en réalisant les étapes (1) à (4) en utilisant des cellules où ladite protéine a été connue comme étant absente, moyennant quoi la présence de ladite substance liée à ladite protéine est déterminée.

9. Procédé selon la revendication 8, dans lequel ladite protéine connue est une protéine récepteur.

10. Procédé selon la revendication 9, dans lequel une souche isolée et établie est utilisée comme cellules dans lesquelles ladite protéine récepteur est exprimée.

11. Procédé selon la revendication 9, dans lequel les cellules forcées artificiellement à exprimer ladite protéine récepteur sont utilisées comme cellules dans lesquelles ledit récepteur est exprimé.

12. Procédé selon la revendication 9, dans lequel des cellules exprimant un récepteur orphelin sont utilisées comme cellules dans lesquelles ladite protéine récepteur est exprimée.

13. Procédé de criblage de cellules ou de tissu, dans lequel une protéine interagissant avec une substance avec une activité connue, est exprimée, comprenant:
la comparaison d'un spectre de masse obtenu par
(1) une étape de mise en contact de ladite substance avec des cellules ou un tissu, où la présence ou l'absence de ladite protéine doit être confirmée, pour lier ladite substance à ladite protéine,
(2) une étape de purification des cellules ou du tissu liant ladite substance,
(3) une étape d'irradiation des cellules ou du tissu purifiés avec un laser, de cette manière ionisant directement ladite substance présente, et
(4) une étape d'analyse de la substance ionisée par spectrométrie de masse à temps de vol avec désorption-ionisation laser assistée par matrice (MALDI-TOF-MS),
avec un spectre de masse obtenu en réalisant uniquement les étapes (2) à (4), sans réaliser l'étape (1) où le ligand est mis en contact avec lesdites cellules ou ledit tissu, moyennant quoi la présence de ladite protéine interagissant avec ladite substance est déterminée.

14. Procédé selon la revendication 13, dans lequel ladite substance est un ligand.

15. Procédé selon la revendication 13 ou 14, dans lequel ladite protéine est une protéine récepteur.
